# EUROPEAN PATENT APPLICATION

(11) **EP 3 243 487 A1**
(43) Date of publication of application: **15.11.2017**
(21) Application number: 17169906.9
(22) Date of filing: 08.05.2017
(51) Int. Cl.: A61F 2/52, A61F 2/50, A61F 2/12

(54) **BREAST PROSTHESIS WITH AIR LAYER**

(30) Priority: 12.05.2016 US 201662335239 P
(71) Applicant: American Breast Care, LP, Marietta, GA 30067 (US)
(72) Inventor: HALLEY, Robert, Atlanta, GA Georgia 30329 (US); WOLLNICK, Amanda, Doraville, GA Georgia 30340 (US)
(74) Representative: Wittmann, Günther

(57) **Abstract**

A breast prosthesis (700) includes a plurality of films, including: a first film (416), a second film (414), a third film (412) and a fourth film (410). An interior weld (610) that seals the second polyurethane film (414), the third polyurethane film (412) and the fourth polyurethane film (410) so as to define an interior chamber (714) between a portion of the second polyurethane film (414) and a portion of the third polyurethane film (412) and a back chamber (710) between a portion of the third polyurethane film (412) and a portion of the fourth polyurethane film (410), the interior chamber (714) being filled with air, the back chamber (710) being filled with a resilient material. A peripheral weld (612) that has a shape of a breast form footprint seals the first film (416), the second film (414), the third film (412) and the fourth film (410). The peripheral weld (612) is spaced apart from and outside of the interior weld (610), so as to define a main chamber (412) between the first film (416) and the second film (414). The main chamber (712) and the back chamber (710) are filled with a second resilient material.

## Description

### BACKGROUND OF THE INVENTION

### 1. Field of the Invention

The present invention relates to breast prostheses and, more specifically, to a lightweight breast prosthesis.

### 2. Description of the Related Art

Many breast prosthesis wearers prefer to wear external breast prostheses that are lightweight. This is due to the fact that the bra provides most of the support for the prosthetic, instead of the chest wall. Standard weight silicone breast prostheses feel heavy and can cause neck, shoulder, and back pain while being worn. Very light, foam based prostheses are available, but they lack the natural feel and drape of natural breast tissue. Additionally, there are silicone breast prostheses with reduced weight available, that have a better feel and drape. The weight reduction is achieved by adding lightweight filler to the silicone, reducing the density. However, only so much of the lightweight filler can be added to the silicone, until the feel and appearance of the prosthesis adversely changes. By having an air filled silicone breast prosthesis, a lighter breast prosthetic can be made without compromising the feel and drape, while also allowing for greater comfort to the wearer.

There are several types of prostheses that are hollow or air-filled. One type is produced by filling a prosthesis mold completely with room temperature vulcanizing plastic. The plastic at the surface of the mold cures before the interior. After a certain time, the partially cured prosthesis is removed from the mold, and a cut is made on the surface. The uncured plastic is then squeezed out of the molded piece, resulting in a hollow prosthetic. This method results in significant material waste.

Other types of breast prosthesis use fillable air bladders, often with the intent of allowing adjustability for fit by the wearer, not necessarily weight reduction. However, manufacturing of such products is complex in regards to placement of the air bladder(s). Secondly, since the air bladder fill volume is allowed to be customized, there are hard port(s) along the bladder(s) for the wearer to attach a hand pump and fill with the desired amount of air. Having a hard port or nozzle could create discomfort while being worn, especially if it were to rub against sensitive post-mastectomy scar tissue. Additionally, the placement of the ports or nozzles limits any additional contours, such as ridges or bumps for air flow that could be molded onto the back of the prosthesis.

Another type of breast prosthesis involves molding a front, shell shape piece out of elastomeric material. Then a second, flat back molded piece is made out of elastomeric material and glued to the front piece. This manufacturing process requires multiple molds and leaves the elastomeric material surface exposed. Many elastomeric materials are lightly tacky and pick up unwanted lint and dirt. Also, prosthesis wearers prefer the feel of gel filled prostheses over rubber constructions.

A common complaint about breast prostheses is that their weight can result in discomfort when worn over long periods.

Therefore, there is a need for a lightweight breast prosthesis.

### SUMMARY OF THE INVENTION

The disadvantages of the prior art are overcome by the present invention which, in one aspect, is a breast prosthesis that includes an outer first layer, a middle second layer and an inner third layer. The outer first layer includes a first material that has a first firmness. The first firmness allows for a 20 mm to a 25 mm penetration by a cone penetrometer. The first layer has a shape corresponding to a shape of a breast form. The middle second layer is disposed adjacent to the first layer and includes a second material that has a second firmness that is greater than the first firmness. The inner third layer is disposed adjacent to the second layer opposite from the first layer and includes a third material that has a third firmness that is less than the second firmness.

In another aspect, the invention is a breast form prosthesis that includes four films that are welded together at a single weld to form three chambers having a shape of a breast form footprint. The three chambers include: a first chamber, a second chamber and a third chamber. An outer first layer is disposed in the first chamber and includes a first material that has a first firmness. The first layer has a shape corresponding to a shape of a breast form. The first material includes a silicone gel with a first concentration of a methyl hydrosiloxane polymer cross-linker. The middle second layer is disposed adjacent to the first layer in the second chamber and includes a second material that has a second firmness that is greater than the first firmness. The second material includes a silicone gel with a second concentration, greater than the first concentration, of a methyl hydrosiloxane polymer cross-linker. The inner third layer is disposed in the third chamber and adjacent to the second layer opposite from the first layer. The inner third layer includes a third material that has a third firmness that is less than the second firmness. The third material includes a silicone gel with a third concentration, less than the second concentration, of a methyl hydrosiloxane polymer cross-linker.

In another aspect, the invention is a method of making a breast prosthesis, in which four flexible films are welded together with a single weld so as to form a breast form envelope. The breast form envelope includes a first chamber, a second chamber and a third chamber. Each chamber has a shape of a breast form footprint. The breast form envelope is placed into a mold having a shape that is complementary in shape to a breast form. A first material is injected into the first chamber. The first material has a first firmness that allows for a 20 mm to a 25 mm penetration by a cone penetrometer when cured. A second material is injected into the second chamber. The second material has a second firmness when cured. The second firmness is greater than the first firmness. A third material is injected into the third chamber. The third material has a third firmness when cured. The third firmness is less than the second firmness. The mold, the breast form envelope, the first material, the second material and the third material are heated to a temperature sufficient to cure the first material to the first firmness, the second material to the second firmness and the third material to the third firmness.

In another aspect, the invention is a breast prosthesis that includes a plurality of films, including: a first film, a second film, a third film and a fourth film. An interior weld seals the second film to the third film so as to define an interior chamber between a portion of the second film and a portion of the third film. The interior chamber is filled with a gas. A peripheral weld that has a shape of a breast form footprint seals the first film, the second film, the third film and the fourth film. The peripheral weld is spaced apart from and outside of the interior weld, so as to define a first chamber between the first film and the second film. The first chamber is filled with a first resilient material. The peripheral weld also defines a second chamber between the third film and the fourth film. The second chamber is filled with a second resilient material.

In another aspect, the invention is a prosthetic breast form that includes a plurality of films, including: a first polyurethane film, a second polyurethane film, a third polyurethane film and a fourth polyurethane film. An interior weld seals the second polyurethane film, the third polyurethane film and the fourth polyurethane film so as to define an interior chamber between a portion of the second polyurethane film and a portion of the third polyurethane film and a back chamber between a portion of the third polyurethane film and a portion of the fourth polyurethane film. The interior chamber is filled with air and the back chamber is filled with a resilient material. A peripheral weld having a shape of a breast form footprint seals the first polyurethane film, the second polyurethane film, the third polyurethane film and the fourth polyurethane film. The peripheral weld is spaced apart from and outside of the interior weld so as to define a main chamber between the first polyurethane film and the second polyurethane film. The main chamber is filled with a resilient material.

In another aspect, the invention is a method of making a breast prosthesis, in which a second film is welded to a third film along an interior weld so that the interior weld defines an interior chamber that is accessible by a first sprue. A first film is placed adjacent to and above the second film and a fourth film is placed adjacent to and below the third film. The first film, the second film, the third film and the fourth film are welded along a peripheral weld that has a shape of a breast form footprint and that is spaced apart from and outside of the interior weld so as to define a first chamber between the first film and the second film that is accessible by a second sprue. The peripheral weld also defines a second chamber between the third film and the fourth film that is accessible by a third sprue. The first chamber is filled with a first material through the second sprue and then the second sprue is sealed. The second chamber is filled with a second material through the third sprue, thereby generating an uncured unit, and then the third sprue is sealed. The uncured unit is placed in a mold having the shape of a breast. A predetermined amount of a gas is injected into the interior chamber through the first sprue and then sealing the first sprue is sealed. The mold is placed into a curing environment and at least one of the first material and the second material is cured.

In yet another aspect, the invention is a method of making a breast prosthesis, in which a second film, a third film and a fourth film are welded along an interior weld so that the interior weld defines an interior chamber defined between the second film and the third film that is accessible by a first sprue. The interior weld also defines a back chamber between the third film and the fourth film that is accessible by a third sprue. A first film is place adjacent to and above the second film. The first film, the second film, the third film and the fourth film are welded along a peripheral weld that has a shape of a breast form footprint and that is spaced apart from and outside of the interior weld. The peripheral weld defines a main chamber between the first film and the second film that is accessible by a second sprue. The main chamber is filled with a first material through the second sprue and then the second sprue is sealed. The back chamber is filled with a second material through the third sprue, thereby generating an uncured unit, and then the third sprue is sealed. The uncured unit is placed in a mold having the shape of a breast. A predetermined amount of a gas is injected into the interior chamber through the first sprue and then the first sprue is sealed. The mold is placed into a curing environment and at least one of the first material and the second material is cured.

These and other aspects of the invention will become apparent from the following description of the preferred embodiments taken in conjunction with the following drawings. As would be obvious to one skilled in the art, many variations and modifications of the invention may be effected without departing from the spirit and scope of the novel concepts of the disclosure.

### BRIEF DESCRIPTION OF THE FIGURES OF THE DRAWINGS

**FIG. 1** is a front elevational view of one embodiment of a three layer breast prosthesis.
**FIG. 2** is a cross-sectional view of the breast prosthesis shown in FIG. 1 taken along line 2-2.
**FIGS. 3A-3F** are a series of schematic drawings showing one method of making a breast prosthesis.
**FIG. 4A** is a front elevational view of one embodiment of a breast form prosthesis.
**FIG. 4B** is a cross-sectional view of the breast form prosthesis shown in FIG. 1, taken along line 4B-4B.
**FIG. 5A** is a side elevational view of two inner films used in making the breast form prosthesis shown in FIG. 1.
**FIG. 5B** is a side elevational view of four films used in making the breast form prosthesis shown in FIG. 1.
**FIG. 5C** is a plan view of one inner film with an air chamber weld.
**FIG. 5D** is a plan view of one outer film with an outer prosthesis weld.
**FIGS. 6A** and **6B** are side schematic views showing an order of filling the chambers defined by the films.
**FIG. 6C** is a side schematic view showing one method of curing the gels in the chambers defined by the films.
**FIG. 7** is a side schematic view of an embodiment of a breast form prosthesis having a textured back.
**FIG. 8A** is a back elevational view of one embodiment employing an air chamber.
**FIG. 8B** is a cross sectional view of the embodiment shown in FIG. 10A, taken along line 8B-8B.
**FIG. 9A** is a side schematic view of three films used in making the embodiment shown in FIG. 10A demonstrating placement of the interior weld.
**FIG. 9B** is a side schematic view of four films used in making the embodiment shown in FIG. 10A demonstrating placement of the exterior weld.
**FIG. 10A** is a side schematic view showing injection of silicon into the back chamber and the main chamber.
**FIG. 10B** is a side schematic view showing injection of air into the interior chamber.

### DETAILED DESCRIPTION OF THE INVENTION

A preferred embodiment of the invention is now described in detail. Referring to the drawings, like numbers indicate like parts throughout the views. Unless otherwise specifically indicated in the disclosure that follows, the drawings are not necessarily drawn to scale. As used in the description herein and throughout the claims, the following terms take the meanings explicitly associated herein, unless the context clearly dictates otherwise: the meaning of "a," "an," and "the" includes plural reference, the meaning of "in" includes "in" and "on."

U. S. Patent Nos. 4,247,351 and 4,249,975 disclose prostheses of silicone gel encased in polyurethane film and, therefore, are incorporated herein by reference. U.S. Patent Nos. 4,950,291, 5,895,423, 5,922,023 and 7,753,954 disclose multi-chambered breast prosthesis and methods of making the same and are also, therefore, incorporated herein by reference.

One embodiment of a breast prosthesis includes three layers of silicone gel enclosed in polyurethane film. The shape of the prosthesis conforms to the shape of a female breast. The layers of silicone are arranged such that a layer of soft conformable silicone is closest to the chest of the wearer and can conform to any irregularities in the chest. The middle layer of silicone is firm and provides support for the prosthesis, and the third layer of silicone positioned in the front of prosthesis (furthest away from the body) is soft and provides a natural drape to the prosthesis, which gives the wearer's chest a natural look and a symmetric appearance.

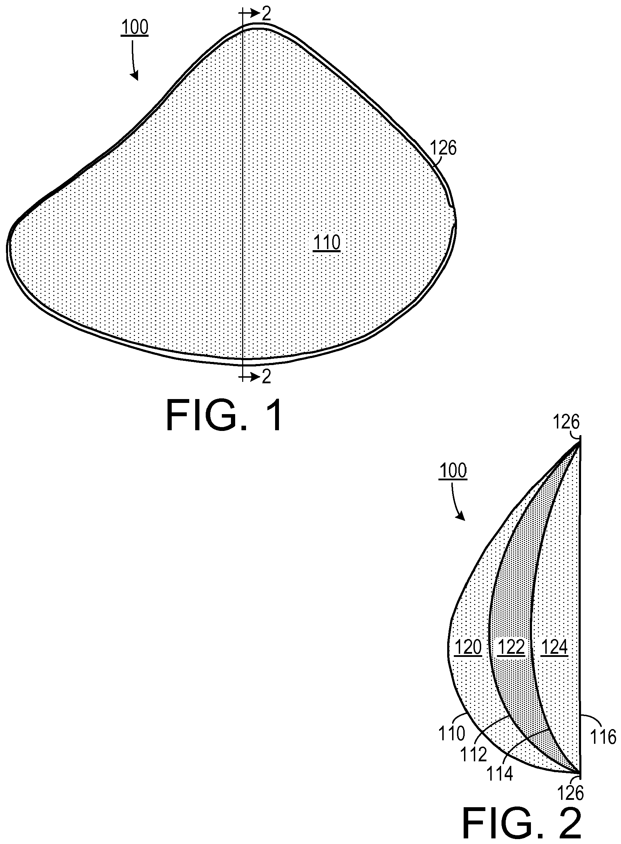

As shown in FIGS. 1 and 2, one embodiment of a three layer breast prosthesis **100** includes an exterior layer **120** that is relatively soft, a middle layer **122** that is relatively firm and an interior layer **124** that is also relatively soft. Each of the layers, **120, 122** and **124,** typically include a silicone gel with the middle layer **122** including a higher concentration of cross-linker to give it additional firmness. The layers are held in flexible chambers that would typically include polyurethane film (typically of about 60 micron to 90 micron thickness) that are sealed together along their periphery along a single weld **126.**

In one embodiment, a first polyurethane film **110,** a second polyurethane film **112,** a third polyurethane film **114** and a fourth polyurethane film **116** are welded together along a single weld **126** to form three chambers. The single weld **126** has the footprint of a breast form. The first material of the first layer **120** is placed in a first of the three chambers. The second material of the second layer **122** is placed in a second of the three chambers. The third material of the third layer **124** is placed in a third of the three chambers.

The first material, the second material and the third material can each include silicone gels. In one embodiment, the silicone gels include a two-component addition-cure silicone gel composition that includes a first combination, including a vinyl polymer, a silicone oil and a cross-linker, and a second combination, including a vinyl polymer, a silicone oil and a catalyst (such as a platinum and silicone complex). (One example of suitable silicon gels include prosthesis gels manufactured by Wacker Chemie GmbH.) A typical cross-linker includes a methyl hydrosiloxane polymer.

In one embodiment the exterior layer **120** and the interior layer **124** have a firmness that allows for a 20 mm to a 26 mm penetration by a cone penetrometer when cured and the middle layer **122** has a firmness that allows for a 10 mm to a 13 mm penetration by a cone penetrometer when cured. In one embodiment, micro-spheres can be added to one or all of the silicone gels to reduce the weight of the resulting prosthesis **100.** Similarly, pigments can be added to give the prosthesis a natural look.

In one experimental embodiment, all silicone gel penetrations were determined on a Humboldt H1200 penetrometer. The probe employed a 65 mm diameter cone (the dimensions corresponded to part number 18-0122 from Petrolab Company) and the total probe weight was 24.9 g.

This breast prosthesis **100** provides a soft back layer **124** that conforms to the chest of the wearer and also a soft front layer **120** that provides a natural drape and fit. The middle layer **122** has sufficient firmness to maintain the shape of the breast form during regular use.

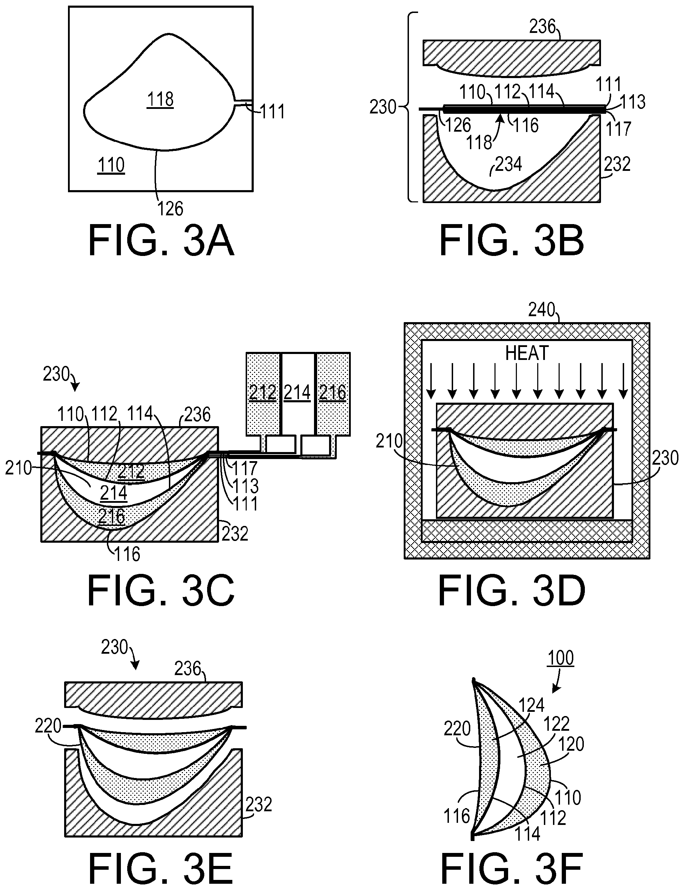

In one embodiment of a method of making a breast form, as shown in FIGS. 3A-3F, four sheets of polyurethane film (**110, 112, 114** and **116**) are welded together along a single weld **126** thereby forming an envelope **118** that includes three different chambers and that has a shape corresponding to a desired breast form. The breast form can be a-symmetric (as in the example shown in FIG. 1) or it can be symmetric. The weld includes a corresponding three sprue inlets (**111, 113** and **117**) that open the chambers to the outside. The envelope **118** is placed in a mold **230** including a front portion **232** and a back portion **236.** The mold **230** defines a cavity **234** having a shape that is complementary to the desired shape of a breast form.

Once in the mold **230,** the first material **212** is injected into the first chamber through the first sprue **111,** the second material **214** is injected into the second chamber through the second sprue **113** and the third material **216** is injected into the third chamber through the third sprue **117.** This forms an uncured breast form **210,** from which any air is removed. The sprues (**111, 113** and **117**) are sealed and the mold **230** with the uncured breast form **210** therein is placed in an oven **240** and heated to a sufficient temperature for a sufficient amount of time to cure the first material **212,** the second material **214** and the third material **216,** thereby forming a cured breast form **220.** The exact temperature and time depend on the specific mixture of silicone gel used; however, it can readily be determined from the manufacturer's data sheet. The cured breast form **220** is removed from the oven **240,** is allowed to cool, removed from the mold **230** and any excess film is trimmed away from the weld, resulting in a breast prosthesis **100.**

The final prosthesis **100** includes a layer **124** closest to the patient that is soft and conforms to the chest of the wearer for comfort. The middle layer **122** is firm and provides a stable structure to the prosthesis. The front layer **120** is soft enough to provide a natural drape to the prosthesis **100** so as to give it an appearance of natural breast tissue.

One embodiment of a three-layer external breast prosthesis includes front layer that is filled with elastomeric material to mimic the shape of the breast, a middle layer of air, and a back layer filled with elastomeric material. The back layer could be flat or molded with ridges or bumps to allow for improved air flow behind the prosthesis, resulting in an even more comfortable wearing experience. Each layer of elastomeric material and the air is enveloped in a wall of polyurethane film. The walls of polyurethane film are welded together along a peripheral edge. This is what joins the three layers of materials together. There are additional inner film welds to control the distribution of elastomeric material and air.

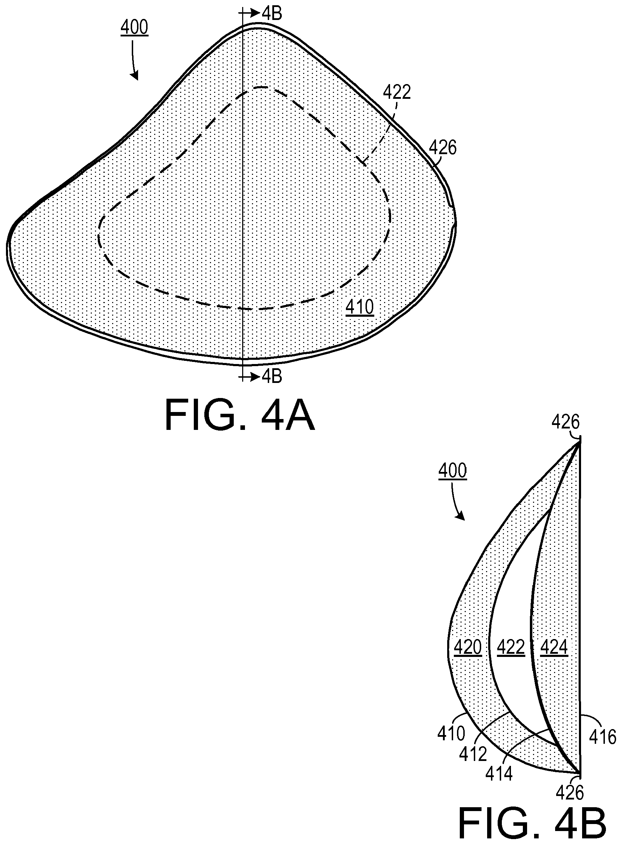

As shown in FIGS. 4A and 4B, one embodiment of a prosthetic breast form **400** includes a first film **410,** a second film **412,** a third film **414,** and a fourth film **416.** These four films would typically include a thermoplastic such as polyurethane. The second film **412** and the third film **414** are welded together to form an interior chamber **422.** The first film **410** is welded to the second film **412** to form a first resilient material-filled chamber **420** and the fourth film **416** is welded to the third film **414** to form a second resilient material-filled chamber **424.** The first resilient material and the second resilient material can include an uncured silicone gel, a cured silicone rubber, a silicone agglomerate putty and the like. The four films are welded to each other at a peripheral weld **426.** U.S. Patent No. 8,926,698, issued to Wollnick et al., discloses methods and compositions for making silicone agglomerate putty and is therefore incorporated herein by reference. In certain embodiments, the interior layer **422** can be filled with air

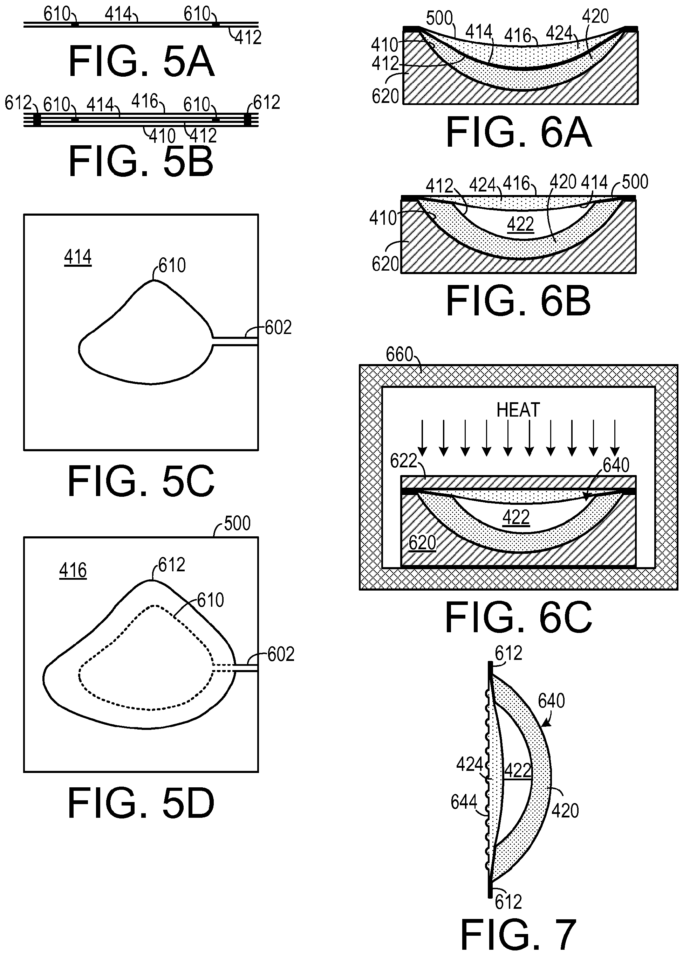

As shown in FIGS. 5A-5D and 6A-6C, several steps are employed in making the embodiment shown in FIGS. 4A-4B, including: Two layers of polyurethane (PU) film **412** and **414** are sealed along an interior weld **610,** which controls the shape of the air layer of the finished prosthesis. Two additional layers of PU film **410** and **416** and welded along an exterior weld **612.** As a result, the films form a blank **500** and define a front chamber **420,** an interior chamber **422** and a back chamber **424.** Each of the chambers is accessible through a different sprue **602.** The blank **500** is placed into a breast shaped mold **620.** An uncured addition-vulcanizing two-component silicone elastomer gel is injected into the front chamber **420** and then the back chamber **424.** A predetermined volume of a gas, such as air, is pumped into the middle chamber **422.** (While air is the preferable gas in many embodiments, it is understood that in certain embodiments other gases, such as nitrogen or helium, might be preferred.) The sprues **602** are closed and sealed to prevent escape of the gas and the silicone elastomer. A back **622** can be placed on the mold **622** and the silicone is then cured by placing the mold **522** in an oven **660.** The resulting breast form **640** is removed from the mold and any extra film is trimmed therefrom.

A back surface texture, as shown in FIG. 7, can be imparted on the back layer **644** by using a mold back **622** with a complimentary-shaped texture. Such a texture, which could include ridges or bumps **644,** can be used to improve air flow, improve comfort to the user and provide a massaging action to the user's chest. In one embodiment, in which it is desirable to have an uncured gel in chamber **424** for comfort reasons, a gel can be injected into chamber **424** after the curing step. Also, microspheres can be mixed into the silicone so as to make a lighter prosthesis. Additionally, an uncured gel can be injected into the back chamber after the curing step, or a non-curing gel may be injected into the back chamber before the curing step.

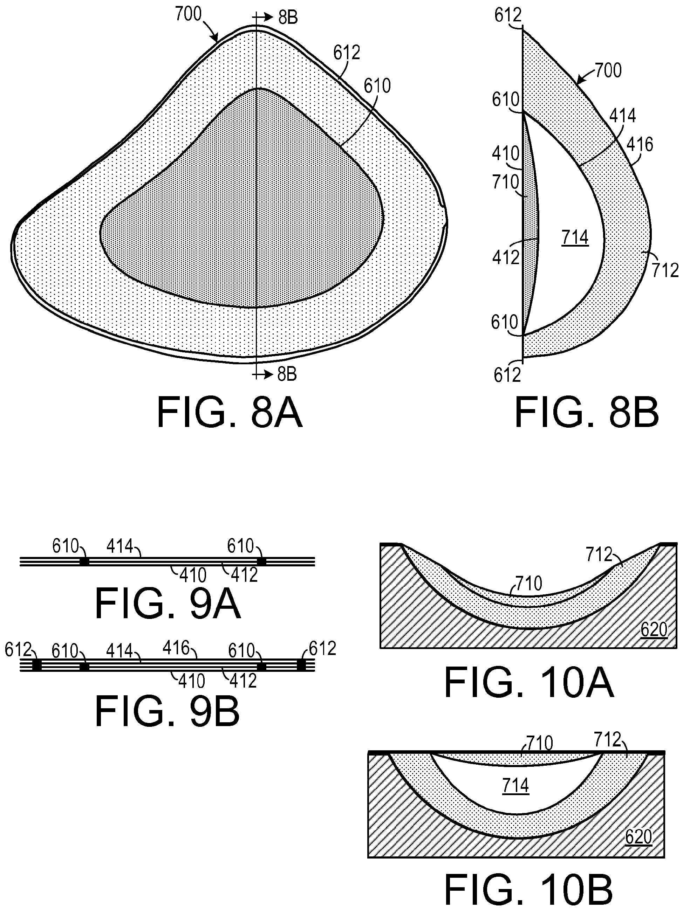

As shown in FIGS. 8A-8B, an alternate embodiment of a breast prosthesis **700** includes a silicone-filled back chamber **710,** an air-filled interior chamber **714** and a silicone-filled main chamber **712.** As shown in FIGS. 9A-9B, the first film **410,** the second film **412** and the third film **414** are welded to each other at an interior weld **610.** This forms a silicone-filled back chamber **710,** an air-filled interior chamber **714,** each of which is accessible through a sprue. A fourth film **416** is added and then all of the films are welded together at a peripheral weld **612.** As shown in FIGS. 10A-10B, a resilient material (such as an uncured silicone) is injected through the sprues into the back chamber **710** and the main chamber **712,** and then the gas is injected into the interior chamber **714.** The resilient material is then cured as described above.

These embodiments have several advantages, including:
- There is no excess material waste to create the inner air cavity. The only material that is used is the amount needed to form the front and back layers.
- The polyurethane film welds are used to adhere the 2-3 layers together. No additional gluing steps are needed.
- The elastomeric material is contained within polyurethane film. This aids in keeping the prosthesis cleaner. It also allows the use of gels, which have a more natural feel and may be more comfortable for some prosthesis wearers.
- The manufacturing process is less complex and resulting in a product that is easier to manufacturer. There's no issue with correct air bladder or nozzle placement. Additionally, separate molds for the front and back layers are not needed.
- The only inlet is at the side and very small and thin, resulting in less discomfort for the wearer.
- The back portion of the prosthesis can be readily molded to have ridges or bumps, to improve air flow while the prosthesis is being worn. There's no need to work around nozzles or inlets.
- The air and elastomeric material distribution is controlled by inner polyurethane film welds versus, multiple and complex molded chambers or air bladders.

The above described embodiments, while including the preferred embodiment and the best mode of the invention known to the inventor at the time of filing, are given as illustrative examples only. It will be readily appreciated that many deviations may be made from the specific embodiments disclosed in this specification without departing from the spirit and scope of the invention. Accordingly, the scope of the invention is to be determined by the claims below rather than being limited to the specifically described embodiments above.

## Claims

1. A prosthetic breast form, comprising:
(a) a plurality of films, including: a first polyurethane film (416), a second polyurethane film (414), a third polyurethane film (412) and a fourth polyurethane film (410);
(b) an interior weld (610) that seals the second polyurethane film (414), the third polyurethane film (412) and the fourth polyurethane film (410) so as to define an interior chamber (714) between a portion of the second polyurethane film (414) and a portion of the third polyurethane film (412) and a back chamber (710) between a portion of the third polyurethane film (412) and a portion of the fourth polyurethane film (410), the interior chamber (714) being filled with air, the back chamber (710) being filled with a resilient material; and
(c) a peripheral weld (612) having a shape of a breast form footprint that seals the first polyurethane film (416), the second polyurethane film (414), the third polyurethane film (412) and the fourth polyurethane film (410), the peripheral weld (612) spaced apart from and outside of the interior weld (610), so as to define a main chamber (712) between the first polyurethane film (416) and the second polyurethane film (414), the main chamber (712) being filled with a resilient material.

2. The prosthetic breast form of Claim 1, wherein the resilient material comprises a material selected from a list of materials consisting of: an uncured silicone gel, a cured silicone rubber, a silicone agglomerate putty, and combinations thereof.

3. The prosthetic breast form of Claim 1, wherein the fourth polyurethane film defines a selected one of ridges or bumps.

4. A method of making a breast prosthesis, comprising the steps of:
(a) welding a second film, a third film and a fourth film along an interior weld so that the interior weld defines an interior chamber defined between the second film and the third film that is accessible by a first sprue and so that the interior weld also defines a back chamber between the third film and the fourth film that is accessible by a third sprue;
(b) placing a first film adjacent to and above the second film;
(c) welding the first film, the second film, the third film and the fourth film along a peripheral weld that has a shape of a breast form footprint and that is spaced apart from and outside of the interior weld, so as to define a main chamber between the first film and the second film that is accessible by a second sprue;
(d) filling the main chamber with a first material through the second sprue and then sealing the second sprue;
(e) filling the back chamber with a second material through the third sprue, thereby generating an uncured unit and then sealing the third sprue;
(f) placing the uncured unit in a mold having the shape of a breast;
(g) injecting a predetermined amount of a gas into the interior chamber through the first sprue and then sealing the first sprue; and
(h) placing the mold into a curing environment and curing at least one of the first material and the second material.

5. The method of Claim 4, wherein the gas comprises air.

6. The method of Claim 4, wherein at least one of the first material and the second material comprises a silicone gel.

7. The method of Claim 4, wherein at least one of the first material and the second material comprises a silicone agglomerate putty.

8. The method of Claim 4, further comprising the step of imparting at least a selected one of ridges or bumps onto the fourth film prior to the curing step.
